**Europäisches Patentamt**

**European Patent Office**

**Office européen des brevets**

(11) Publication number: **0 338 363 B1**

## EUROPEAN PATENT SPECIFICATION

(45) Date of publication of patent specification: **15.03.95**

(51) Int. Cl.6: **A61N 1/37**

(21) Application number: **89106278.8**

(22) Date of filing: **10.04.89**

(54) Configuration programming of an implantable pacemaker.

(30) Priority: **19.04.88 US 183197**

(43) Date of publication of application:
**25.10.89 Bulletin 89/43**

(45) Publication of the grant of the patent:
**15.03.95 Bulletin 95/11**

(84) Designated Contracting States:
**DE FR GB IT NL SE**

(56) References cited:
**EP-A- 0 114 679         EP-A- 0 308 536**
**GB-A- 2 026 870         US-A- 4 140 131**
**US-A- 4 402 322         US-A- 4 549 548**
**US-A- 4 606 349**

(73) Proprietor: **Pacesetter AB**
**Röntgenvägen 2**
**S-171 95 Solna (SE)**

(72) Inventor: **Hafelfinger, Werner**
**25389 Fortuna Drive**
**Valencia, CA 91355 (US)**
Inventor: **Sholder, Jason A.**
**21037 Cantara Street**
**Canoga Park, CA 91304 (US)**
Inventor: **King, Murray P.**
**11528 Coralberry Court**
**Moorpark, CA 93021 (US)**
Inventor: **Duncan, James L.**
**225 Hepplewhite Drive**
**Alpharetta, GA 30201 (US)**

(74) Representative: **Rees, David Christopher**
**Kilburn & Strode**
**30 John Street**
**London WC1N 2DD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to implantable pacemakers and, more particularly, to implantable pacemakers having the capability of being programmed to operate in either a unipolar or bipolar mode of operation, and including the capability of having either unipolar or bipolar leads connected thereto.

BACKGROUND OF THE INVENTION

The technology of cardiac pacemakers has developed a high level of sophistication of system performance. The current generation of cardiac pacemakers incorporates microprocessors and related circuitry to sense and stimulate heart activity under a variety of physiological conditions. These pacemakers may be programmed to control the heart in correcting or compensating for various heart abnormalities which may be encountered in individual patients. A background description of modern cardiac pacemaker technology is set forth in U.S. Patent 4,712,555.

In order to efficiently perform its function as a pump, the heart must maintain a natural AV synchrony. The term "AV synchrony" relates to the sequential timing relationship that exists between the contractions of the atria and the ventricles. In a given heart cycle or beat, these contractions are typically manifest or measured by sensing electrical signals or waves that are attendant with the depolarization of heart tissue, which depolarization immediately precedes (and for most purposes can be considered concurrent with) the contraction of the cardiac tissue. These signals or waves can be viewed on an electrocardiogram and include a P-wave, representing the depolarization of the atria; the QRS wave (sometimes referred to as an R-wave, the predominant wave of the group), representing the depolarization of the ventricles; and the T-wave, representing the repolarization of the ventricles. (It is noted that the atria also are repolarized, but this atrial repolarization occurs at approximately the same time as the depolarization of the ventricles; and any electrical signal generated by atrial repolarization is generally minute and is masked out by the much larger QRS-wave on the electrocardiogram.)

Thus, it is the P-QRS-T cycle of waves that represents the natural AV synchrony of the heart. These waves, including the time relationships that exist therebetween, are carefully studied and monitored through conventional ECG techniques whenever the operation of the heart is being examined.

It is a primary goal of most multiple-mode, demand-type, cardiac pacemakers to maintain an AV synchrony for damaged or diseased hearts that are unable to do so on their own. It is noted, however, that sometimes it may not be necessary for a particular patient to maintain AV synchrony; hence, the pacemaker may be programmed to operate in a VVI mode of operation (which mode is described more fully below), or only a single chamber pacer may be implanted in the patient for the purpose of controlling one heart chamber. In either case, a demand-type mode of operation is usually employed. A demand-type pacemaker is one that provides a stimulation pulse only when the heart fails to produce a natural depolarization on its own within a prescribed escape interval. In a dual chamber pacemaker, this is realized by placing electrodes in both the right atrium and right ventricle of the heart. (In a single chamber pacemaker, electrodes are placed only in one heart chamber.) These electrodes are coupled through intravenous and/or epicardial leads to sense amplifiers housed in an implanted pacemaker. Electrical activity occurring in these chambers can thus be sensed. When electrical activity is sensed, the pacemaker assumes that a depolarization or contraction of the indicated chamber has occurred. If no electrical activity is sensed within a prescribed time interval, typically referred to as an atrial or ventricular escape interval, then a pulse generator, also housed within the pacemaker housing, generates a stimulation pulse that is delivered to the indicated chamber, usually via the same lead or electrode as is used for sensing. This stimulation pulse causes or forces the desired depolarization and contraction of the indicated chamber to occur. Hence, by first sensing whether a natural depolarization occurs in each chamber, and by second stimulating at controlled time intervals, each chamber with an external stimulation pulse in the absence of a natural depolarization, the AV synchrony or other desired response of the heart can be maintained. Thus, with a demand pacer, the heart will either beat on its own (without stimulation from the pacemaker) at a rate that is at least just slightly faster than the stimulation rate defined by the escape interval, or the heart will be stimulated by the pacer at a rate controlled by the escape interval. The stimulation rate provided by the pacemaker is typically referred to as the "programmed rate."

Pacemakers have come to be classified according to type and functional mode of operation. For several years, a three-letter code has been used to describe the various types of pacemakers. In accordance with this code, the letter in the first position has been used to indicate the chamber being paced. Thus, V represents the ventricle, A represents the atrium, and D indicates that both the atrium and the ventricle are being paced. The letter in the second position for this code indicates the chamber being sensed. As with the chamber being paced, V, A and D when in the second position of the code indicate the chamber being sensed is the ventricle, the atrium or both the atrium and ventricle, respectively. In addition, O represents the lack of sensing. The letter in the third position in this three-letter code signifies the mode of response of the pacemaker. In this position, T represents triggered response, I represents inhibited response, D represents double response (atrial triggered and ventricle inhibited or atrial triggered/inhibited and ventricle inhibited). O indicates none and R indicates reversed (where the pacemaker is activated by a fast heart rate but does not respond to a slow heart rate--in other words, a specialized antiarrhythmic pacemaker).

As noted, most pacemakers include a sensor circuit that looks for electrical signals from spontaneous heart activity. On detection of such activity, the pacemaker stimulation action is modified, depending upon the functional mode or type of pacemaker. For example, in the VVI mode (ventricle paced and sensed, response inhibited mode), sensing of heart activity under certain time restrictions is interpreted as normal heart activity such that the stimulating action is inhibited.

The discussion thus far has followed the assumption that a pacemaker and its associated circuitry operate without malfunction. By the very nature of man-made devices, such is not always the case. Whereas electronic circuitry can be, and is, incorporated within the pacemaker itself for exercising or testing various circuit components, such as the status of battery power sources, and the effectiveness of various amplifiers, waveform shaping stages and the like, it is often more difficult to test the integrity of the leads and implanted electrodes to which the pacemaker is coupled in order to verify that such leads and electrodes can function to allow for the desired pacing operation.

At the implanting of the pacemaker and electrode system, minor damage is sometimes incurred which may affect the system's electrical insulation. This type of damage may go undetected and be without present effect on the implanted system, but the condition may manifest itself after extended time in service. When a breakdown or significant degradation of the pacemaker lead insulation occurs, it can have serious or even disastrous results, depending upon whether or not the breakdown is of a catastrophic nature. Various types of lead damage may produce different types of failure or degradation. For example, an insulation defect on the stimulation electrode shunts the energy intended for the heart to some other point. A lead breakage can in some environmental situations temporarily or permanently reduce the stimulation output, sometimes drastically. Another type of detectable error relates to the failure of the electrode tip to be in proper contact with the heart wall.

Particular methods and apparatus for scanning the implanted leads of a pacemaker system to determine lead impedance and to detect abnormalities which may signal degradation and impending failure of pacemaker leads may include a scanning system for measuring the output energy delivered to the stimulation circuit during pacing and determining the lead impedance from that measurement. The thus-determined lead impedance is compared with a moving average of the measured parameter and any deviation from that average by more than a predetermined amount is considered an anomaly. Three such anomalies in succession result in an event being counted in a first event counter for future consideration by a doctor during a patient checkup or the like. The system also monitors sensed heart signals and counts as a notable event any deviations in slope of the heart signal by more than a predetermined amount. These latter events are counted in a second counter to provide information for future reference. Thus, the system makes measurements of both the pacing and sensing leads in a dual chamber lead system or makes measurements of an implanted lead under two different conditions in a single chamber system.

U.S. Patent 4,140,131 (Dutcher, et al.) discloses arrangements for detecting impedance level (either too high in the case of an open circuit or too low in the case of a short circuit) and voltage level of the power source so that a warning may be given the patient. The disclosed method is but one example of ways of measuring lead impedance which might be employed in connection with the present invention. Basically, the lead resistance is considered as part of a voltage divider circuit and lead resistance is calculated from knowledge of impressed voltage and voltage across a known series resistor.

U.S. Patent 4,549,548 (Wittkampf, et al.) discloses a programmable pacemaker system in which the selection of lead electrodes to which pacemaker output may be connected is changed during each pacer cycle to optimize the choice of unipolar and bipolar operation for given pacemaker events. According to the patent, the selection of unipolar or bipolar mode of operation is based on a determination for monitoring the amplitude of sensed heartbeat signals to determine whether the sensing operation would be performed better in the unipolar or the bipolar mode. This is directed to a determination of heart performance vis-a-vis the leads involved so as to control the selection of unipolar or bipolar sensing.

As indicated by U.S. Patent 4,606,349 (Livingston, et al.), it is known in the pacemaker art to provide a pacemaker that can be programmably switched to operate in either a unipolar or bipolar mode of operation.

While the Livingston, et al. patent suggests that a bipolar lead always be connected to the pacemaker, which lead is then used in either a bipolar (tip-to-ring) or unipolar (tip-to-case) mode of operation, there are situations where it is desirable to use a unipolar lead. A problem thus arises when such a pacemaker has a unipolar lead connected thereto but has been programmed to operate in the bipolar mode.

Similarly, if a bipolar lead is being used in a bipolar mode of operation, and if one of the conductors breaks, the pacemaker is not able to continue operation without being reprogrammed. Unfortunately, such reprogramming can only be performed by a doctor after he has diagnosed that the lead conductor has broken. Then, if it is the ring conductor that has broken, the pacemaker could be reprogrammed to operate in a unipolar mode of operation using the existing implanted broken bipolar lead as a unipolar lead (tip-to-case). If it is the tip conductor that has broken, the pacemaker may, in some situations, be reprogrammed to operate in a unipolar ring-to-case mode of operation. In either event, it takes careful diagnosis and reprogramming before the pacemaker can be rendered operational. For many patients, the loss of pacemaker operation for the time period between breaking of the conductor and reprogramming of the pacemaker could present a serious health risk.

What is needed, therefore, is a pacemaker that can be programmed to operate in either a bipolar or unipolar mode of operation, that can receive either bipolar or unipolar leads, and that automatically prevents a programming configuration inconsistent with the lead used. Further, there is a need to have such a pacemaker that automatically responds to a broken conductor of a bipolar lead so as to preserve whatever pacemaker operation may be available using the remaining intact conductors of the bipolar lead. The present invention addresses these and other needs.

## SUMMARY OF THE INVENTION

The present invention is intended for use with an implantable pacemaker that has configuration programming capabilities as well as unipolar/bipolar lead interchangeability (meaning that either unipolar or bipolar operation may be programmed and either unipolar or bipolar leads may be inserted into the pacemaker connector block).

Basically, a first feature of the invention provides a special monitoring circuit within the pacemaker that performs a lead impedance measurement whenever the operating configuration of the pacemaker is programmably changed, e.g., from bipolar to unipolar or vice versa. From this measurement, a determination is made as to whether the correct impedance is present for the existing operating configuration. If the expected impedance is not measured, then a different configuration measurement is made according to a predefined sequence until a correct impedance value is measured. That value is then used to set the pacemaker configuration accordingly.

To illustrate how this feature of the invention works, consider a pacemaker programmed to operate in the bipolar mode but to which a unipolar lead has been connected. Because the pacemaker is programmed to operate in the bipolar configuration, it makes its initial impedance measurement from tip to ring. However, because a unipolar lead has no ring, such a measurement would yield a very large impedance, certainly not within the expected range of 200-1500 ohms. Hence, the pacemaker next makes a tip-to-case impedance measurement. This measurement, assuming a properly paced unipolar lead is present, yields a valid impedance value. This valid measurement signals the configuration circuits of the pacemaker to switch to a unipolar mode of operation. If the tip-to case measurement also yielded an invalid impedance measurement, as might occur for example if a bipolar lead were in place but the tip conductor became broken, then the pacemaker would next make a ring-to-case impedance measurement. If such measurement produced a valid impedance value, then the pacemaker configuration circuits could switch to a unipolar, ring-to-case operation.

As a safety feature, similar impedance checks or measurements would automatically be performed by the pacemaker whenever it detected lack of capture (i.e., failure of the heart chamber to depolarize in response to an applied stimulation pulse). Such measurements could be performed, for example, during a refractory period of the pacemaker. If the lack of capture were caused by a broken conductor within the pacemaker lead, such measurements would quickly identify which conductor had broken, and would then allow the pacemaker to switch to whatever configuration was available to use with any remaining non-broken conductor leads so as to again effectuate capture (and thus allow the pacemaker to fulfill its intended function despite the broken conductor within the lead).

Thus, it is seen that there are three impedance measurements that can be made. These are:
1. tip-to-ring,
2. tip-to-case, and
3. ring-to-case.

If a bipolar configuration has been programmed, then these three measurements are made in the order listed. If a unipolar configuration has been programmed, then measurements 2 and 3 are made in the order listed. Once a correct impedance measurement is found, further impedance measurements are stopped and the configuration of the pacemaker is switched to operate in accordance with the available lead configuration sensed. That is, the pacing configuration of the pacemaker is switched to be the same as the sensing configuration of the pacemaker that yielded a valid impedance measurement.

## BRIEF DESCRIPTION OF THE DRAWINGS

The above and other objects, features and advantages of the present invention will be more apparent from the following more particular description thereof presented in conjunction with the accompanying drawings, wherein:

Fig. 1 is a schematic representation of a dual chamber cardiac pacemaker shown implanted in association with a heart for pacing;

Fig. 1A is a schematic representation of a single chamber cardiac pacemaker shown implanted in association with a heart for unipolar lead pacing;

Fig. 2 is a schematic block diagram of one particular arrangement disclosed in the above-referenced Ekwall application for incorporation in a pacemaker like that shown in Fig. 1; and

Fig. 3 is a functional block diagram showing one particular arrangement in accordance with the present invention.

## DETAILED DESCRIPTION OF THE INVENTION

The following description is of the best presently contemplated mode of carrying out the invention. This description is not to be taken in a limiting sense but is made for the purpose of describing the general principles of the invention. The scope of the invention should be determined with reference to the appended claims.

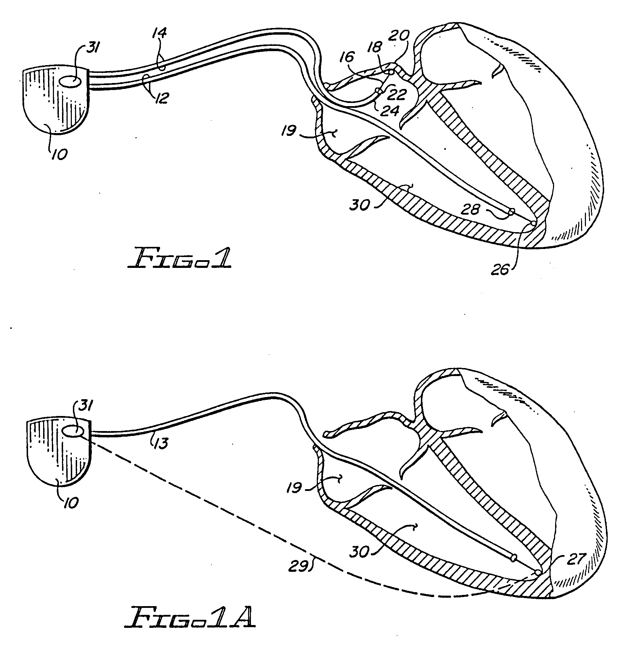

Referring now to Fig. 1, there is shown a simplified representation of one way that an implanted pacemaker 10 may make electrical contact with the heart. Fig. 1 depicts the use of two bipolar leads 12 and 14, each being directed into a separate chamber of the right heart. A bipolar lead comprises a single filar that includes two electrically insulated conductors. For example, the lead 14 includes a first conductor 16 that is electrically connected to a distal tip 18 of the lead. This distal tip is typically placed in a cavity of the right atrium 19 referred to as the atrial appendage 20. A known distance from the distal tip 18 an electrode ring 22 is electrically connected to the other conductor 24 of the bipolar lead 14. Similarly, a distal tip 26 and a conductive ring 28 are associated with the bipolar lead 12 that is placed in the apex of the right ventricle 30. The manner in which the leads 12 and 14 are inserted into the heart, as well as the manner in which the pacemaker 10 is implanted in the body of a patient, are well known in the art. It will be understood that the pacemaker 10 is a programmable pacemaker with the capability of operating in any selected mode and in either a bipolar or unipolar configuration.

The diagram of Fig. 1 may be considered to represent a pacer operating in the VVI mode if the bipolar lead 14 with its associated distal tip 18 and electrode ring 22 is eliminated from the figure so that only the bipolar lead 12 is left with its tip and ring 26, 28 inserted in the right ventricle 30, as shown in Fig. 1.

Fig. 1A is a diagram similar to that of Fig. 1 but showing the pacemaker 10 connected to a single insulated lead 13 which has a tip electrode 27 implanted in the apex of the ventricle 30 for operation as a unipolar pacemaker. Lead 13 is a single conductor, insulated lead with its sole conductor extending at an exposed metal tip 27. The return path for current between the tip electrode 27 and the case of the pacemaker 10 to an exposed region 31 is indicated by the broken line 29. A unipolar pacemaker may be used for both stimulation and sensing, although it may be somewhat more sensitive to interference from external signals such as muscle contraction or electromagnetic interference because of the longer return current path.

A block diagram of a circuit disclosed in the above-identified application of Ekwall is shown in Fig. 2. The lead impedance analyzing circuit 40 is shown comprising a stimulation timing circuit 42 which contains the normal pacemaker timing and logic circuitry. The circuit 40 is coupled to control the actuation of a switch S1 upon the occurrence of a stimulation signal STIM. The source of the pacing output at terminal 46 is a capacitor C1 which is coupled to be charged by a charging circuit 44 and which delivers the pacing pulse to the output 46 through a series capacitor C4 when the switch S1 is closed. Resistor R2 is provided to complete the circuit to capacitor C4 when switch S1 is open.

A sampling stage 50 is coupled to sample capacitor C1 before and after delivery of the pacing pulse. Sampled voltages from the sampling stage 50 are delivered to an analog-to-digital (A/D) converter 52, the output of which in digital form is applied to a stimulating impedance discriminator 54 which contains the circuitry for evaluating changes in lead impedance as a function of the voltage difference between the two levels sampled before and after delivery of a stimulation pulse, corresponding to the following equation:

$$R = Tp/(C1 \; 1n(1-dV/Vo)) \qquad (1)$$

where

R      represents a lead impedance,

Tp      is a proportionality constant,

C1      is the source capacitor for the stimulation pulse,

Vo      is the source voltage, and

dV      is the voltage difference between the sampled voltages.

The stimulating impedance discriminator 54 maintains a moving average of lead impedance measurements, displayed in stage 55, according to Equation (1) and compares each new measurement with that average. If the measurement of lead impedance differs from the moving average by a predetermined value, an associated counter 56 is incremented to count the event as the occurrence of an error.

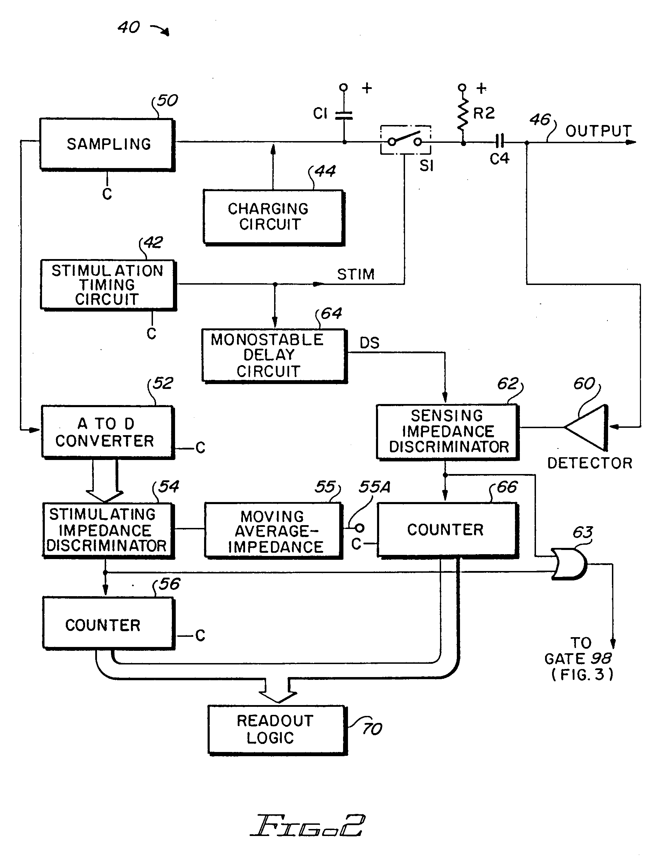

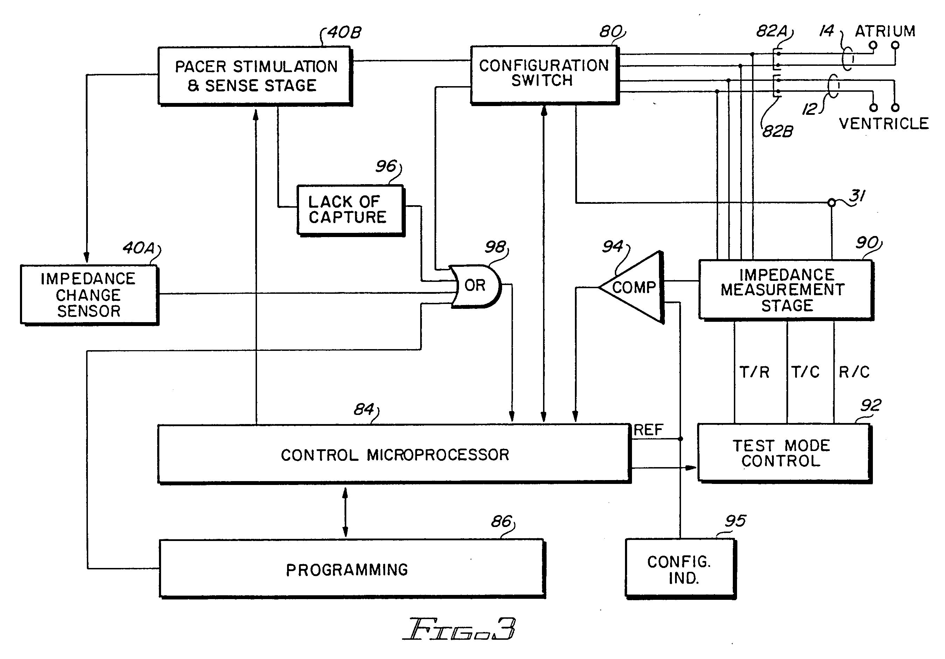

A sensing detector 60 is coupled to the terminal 46 to respond to sensed heart activity. The output of the sensing detector 60 is applied to a sensing impedance discriminator 62 which receives a delayed signal DS from a monostable delay circuit 64 that is triggered by the STIM output of the stimulation timing circuit 42. The output of the sensing impedance discriminator 62 is applied to a second counter 66. The sensing impedance discriminator 62 responds to the slope (slew) of the signal from the sensing detector 60 such that when a rate of change greater than 10 volts per second is detected, the count in the counter 66 is incremented. A readout logic stage 70 is coupled to the outputs of both counter stages 56 and 66 to provide an indication of the number of errors detected by the respective portions (stimulating signal and sensing signal) of the lead impedance analyzing circuit 40. The inputs designated by the letter C indicate connections from the various stages to a system clock (not shown). For use in the present invention, an OR gate 63 is coupled to send a signal to a gate 98 in Fig. 3 upon the occurrence of an event which would increment either counter 56 or 66, thus signaling the circuit of Fig. 3 that a significant departure from normal impedance has been detected.

Fig. 3 is a functional block diagram representing one particular arrangement in accordance with the present invention. The circuit of Fig. 2 is represented in Fig. 3 as comprising the blocks 40A and 40B. A configuration switch 80 is shown connected between pairs of leads 12 and 14 via connectors 82A, 82B and the pacing stimulation and sense stage 40B. Also shown is the connection to the case 31. In conventional fashion, a control microprocessor 84 or equivalent, is coupled to control the pacer stimulation and sense stage 40B and the configuration switch 80. A programming block 86 indicates the capability of programmably controlling the microprocessor 84.

The system of Fig. 3 is shown as further comprising an impedance measurement stage 90 coupled to be controlled by a test mode control stage 92 which in turn is controlled by the microprocessor 84. The impedance measurement stage 90 has connections to each lead in the pairs 12, 14 as well as to the pacer case 31. The output of the impedance measurement stage 90 is directed to a comparator stage 94 which receives a reference signal REF from the microprocessor 84 and is coupled to direct the results of its comparison to the microprocessor 84 for further action.

The impedance measurement stage 90 preferably measures lead impedance during the refractory period. The circuit of Fig. 2 measures impedance from the stimulation pulse and from sensed heart activity. Measurement of lead impedance as a moving average of a group of measurements (e.g., three in succession) is available at terminal 55A of the impedance measurement stage 55 of Fig. 2. If preferred, this may be substituted for the impedance measurement stage 90.

A stage 96 is coupled to the pacer stimulation and sense stage 40B to provide a signal whenever the pacemaker detects lack of capture (i.e., failure of the heart chamber to depolarize in response to an applied stimulation pulse). The output of the lack-of-capture stage 96 is applied to an OR gate 98, as are also signals from the configuration switch 80, impedance change sensor 40A and the programming stage 86. The OR gate 98 applies an initiation signal to the microprocessor 84 upon any one of the following events: (a) receipt of an applied signal from the configuration switch 80 corresponding to the change of connection of any of the leads at the terminals 82A, 82B; (b) a lack-of-capture signal from the stage 96; (c) a significant change of impedance signal from the sensor 40A; and (d) a signal from the programming stage 86 indicating a change in the programmed lead configuration.

An initiation signal from the OR gate 98 causes the microprocessor 84 to actuate the test mode control stage 92 to begin testing the impedance of the leads 12, 14 and the body circuit path to the case 31 in a predetermined sequence and in accordance with the particular lead configuration presently programmed. The impedance measurements performed by the stage 90 are preferably taken during a refractory period of the pacemaker. The three types of impedance measurements -- T/R (tip-to-ring), T/C (tip-to-case) and R/C (ring-to-case) -- are conducted in that order as described hereinabove if a bipolar configuration is programmed. If a unipolar configuration is programmed, only the T/C and R/C measurements are performed. Once a proper impedance measurement is found, the measurement cycle is terminated and the configuration of the switch 80 is changed to correspond to operation in the available lead configuration which is sensed. This new configuration is stored in the microprocessor 84 and is available as an indication from the REF lead at the configuration indicator 95.

Thus, it is seen that the present invention provides a system whereby the pacemaker seeks out, based on load recognition (the impedance load presented by the lead/tissue interface), which lead configuration is operable. If this lead configuration matches the programmed pacemaker configuration, then that is the configuration in which the pacemaker operates. However, if this lead configuration does not match the programmed pacemaker configuration, indicating either an incorrectly programmed pacemaker for the lead used, or a broken lead conductor, then the pacemaker switches its operating configuration, if possible, to a configuration that does match an available lead configuration, thereby allowing the pacemaker to continue to operate.

If a configuration change is automatically initiated by the present invention, then means are provided to signal this fact during the next interrogation of the pacemaker.

Although there have been described above specific arrangements for configuration programming of an implantable pacemaker in accordance with the invention for the purpose of illustrating the manner in which the invention may be used to advantage, it will be appreciated that the invention is not limited thereto. Accordingly, any and all modifications, variations or equivalent arrangements which may occur to those skilled in the art should be considered to be within the scope of the invention as defined in the annexed claims.

## Claims

1.  In an implantable pacemaker (10) wherein the operating configuration may be selectively programmed to be either unipolar or bipolar, a system for ensuring pacemaker operation despite incorrect programming or broken conductor leads (12, 13, 14), said system being characterized by:

    means (90) for measuring lead impedance between a first lead conductor and a second return conductor, said first and second conductors being defined by the selected programmed configuration of said pacemaker (10);

    means (94) for determining whether the value measured by said impedance measuring means (90) is within a preselected range;

    means (84, 92) responsive to said determining means for redefining said first and second conductors according to a preselected sequence whenever an impedance measurement falls outside of said preselected range;

    means (84,92) for repeating the lead impedance measurement using the redefined first and second conductors; and

    means (80) for switching the programmed configuration of the pacemaker (10) to match a configuration defined by the redefined first and second conductors whenever an impedance measurement of the redefined first and second conductors falls within said preselected range.

2.  The system of claim 1 wherein said first and second conductors correspond to a predetermined pair of a group comprising tip (20, 26, 27), ring (22, 28) and case (31) conductors.

3.  The system of claim 2 wherein said preselected sequence comprises tip-to-ring, tip-to-case, and ring-to-case.

4.  The system of claim 2 wherein said pair comprises tip (20, 26, 27) and case (31) conductors and wherein said preselected sequence comprises tip-to-case and ring-to-case.

5.  The system of claim 1 further including means (40 A) for sensing a significant change of lead impedance during normal pacemaker operation and means (84, 98) for initiating said measurement of lead impedance upon detection of said significant change of lead impedance.

6.  The system of claim 5 further including means (80) for initiating said measurement of lead impedance upon the occurrence of a change of leads coupled to the pacemaker (10).

7.  The system of claim 6 further including means (86) for initiating said measurement of lead impedance upon a change in the programmed configuration of said pacemaker (10).

8.  The system of claim 7 further including means (96) for initiating said measurement of lead impedance upon detection of a lack of capture.

9.  The system of claim 1 wherein said determining means (94) comprises a comparator (94) coupled to receive measurement signals from said lead impedance measuring means (90) for comparing said signals with a predetermined reference impedance value corresponding to a programmed configuration.

## Patentansprüche

1. Ein implantierbarer Herzschrittmacher (10), bei dem die Betriebskonfiguration wahlweise so programmiert werden kann, daß sie entweder unipolar oder bipolar ist, mit einem System, das den Herzschrittmacherbetrieb ungeachtet inkorrekter Programmierung oder unterbrochener Elektodenleitungen (12, 13, 14) sicherstellt, wobei dieses System **gekennzeichnet** ist **durch:**

   Mittel (90) zum Messen der Elektrodenvorrichtungsimpedanz zwischen einem ersten Elektrodenleiter und einem zweiten Rückführungsleiter, wobei diese ersten und zweiten Leiter durch die gewählte programmierte Konfiguration des Herzschrittmachers (10) definiert sind;

   Mittel (94) zum Bestimmen, ob der durch die Impedanzmeßmittel (90) gemessene Wert innerhalb eines vorbestimmten Bereiches liegt;

   Mittel (84,92), die auf die Bestimmungsmittel ansprechen, um die ersten und zweiten Leiter entsprechend einer vorgewählten Sequenz neu zu definieren, wann immer eine Impedanzmessung außerhalb des vorbestimmten Bereiches fällt;

   Mittel (84,92) zum Wiederholen der Messung der Elektrodenvorrichtungsimpedanz, wobei die neu definierten ersten und zweiten Leiter verwendet werden; und

   Mittel (80) zum Umschalten der programmierten Konfiguration des Herzschrittmachers (10), um sie der durch die neu definierten ersten und zweiten Leiter definierten Konfiguration anzupassen, wann immer eine Impedanzmessung der neu definierten ersten und zweiten Leiter in den vorbestimmten Bereich fällt.

2. Das System nach Anspruch 1, wobei die ersten und zweiten Leiter mit einem vorbestimmten Paar aus einer Gruppe korrespondieren, die den Elektrodenkopf (20, 26, 27), die Ringelektrode (22, 28) und das Gehäuse (31) enthält.

3. Das System nach Anspruch 2, wobei die vorgewählte Sequenz Elektrodenkopf zu Elektrodenring, Elektrodenkopf zu Gehäuse und Elektrodenring zu Gehäuse enthält.

4. Das System nach Anspruch 2, wobei das Paar von Leitern den Elektrodenkopf(20, 26, 27) und das Gehäuse (31) und die vorgewählte Sequenz Elektrodenkopf zu Gehäuse und Elektrodenring zu Gehäuse enthält.

5. Das System nach Anspruch 1 zusätzlich mit Mitteln (40 A) zum Abfühlen einer signifikanten Änderung der Elektrodenvorrichtungsimpedanz während normalen Herzschrittmacherbetriebs und mit Mitteln (84, 98) zum Starten der Messung der Elektrodenvorrichtungsimpedanz beim Detektieren der signifikanten Änderung der Elektrodenvorrichtungsimpedanz.

6. Das System nach Anspruch 5 zusätzlich mit Mitteln (80) zum Starten der Messung der Elektrodenvorrichtungsimpedanz beim Auftreten eines Wechsels der Elektrodenvorrichtungen, die an den Herzschrittmacher (10) angeschlossen sind.

7. Das System nach Anspruch 6 weiterhin mit Mitteln (86) zum Starten der Messung der Elektrodenvorrichtungsimpedanz bei einem Wechsel der programmierten Konfiguration des Herzschrittmachers (10).

8. Das System nach Anspruch 7 weiterhin mit Mitteln (96) zum Starten der Messung der Elektrodenvorrichtungsimpedanz beim Detektieren fehlender Reizschwellenfindung (capture).

9. Das System nach Anspruch 1, wobei die Bestimmungsmittel (94) einen Komparator(94) aufweisen, der so angeschlossen ist, daß er Meßsignale von den Mitteln (90) zum Messen der Elektrodenvorrichtungsimpedanz empfängt, und diese Signale mit einem vorbestimmten Referenzimpedanzwert, der zu der programmierten Konfiguration korrespondiert, vergleicht.

## Revendications

1. Dans un stimulateur cardiaque (10) implantable dans lequel la configuration de fonctionnement peut être programmée de façon sélective pour être soit unipolaire soit bipolaire, un système pour s'assurer du fonctionnement du stimulateur cardiaque malgré une programmation incorrecte ou une rupture des dérivations (12, 13, 14) conductrices, le système étant caractérisé par:

   un moyen (90) destiné à mesurer l'impédance de dérivation entre un premier conducteur de dérivation et un second conducteur de retour, les premier et second conducteurs étant définis par la configuration programmée sélectionnée du stimulateur cardiaque (10);

   un moyen (94) destiné à déterminer si la valeur mesurée par le moyen (90) de mesure d'impédance se situe à l'intérieur d'une gamme présélectionné ;

un moyen (84, 92) sensible au moyen de détermination pour redéfinir les premier et second conducteurs selon une séquence présélectionnée chaque fois qu'une mesure d'impédance se situe à l'extérieur de la gamme présélectionnée;

un moyen (84, 92) destiné à répéter la mesure d'impédance de dérivation par utilisation des premier et second conducteurs redéfinis; et

un moyen (80) destiné à effectuer une commutation de la configuration programmée du stimulateur cardiaque (10) afin d'adapter une configuration définie par les premier et second conducteurs redéfinis chaque fois qu'une mesure d'impédance des premier et second conducteurs redéfinis se situe à l'intérieur de la gamme présélectionnée.

2. Système selon la revendication 1, dans lequel les premier et second conducteurs correspondent à une paire prédéterminée d'un groupe comprenant des conducteurs d'extrémité (20, 26, 27), d'anneau (22,28) et de boîtier (31).

3. Système selon la revendication 2, dans lequel la séquence présélectionnée est la séquence extrémité-à-anneau, extrémité-à-boîtier, et anneau-à-boîtier.

4. Système selon la revendication 2, dans lequel la paire est constituée des conducteurs d'extrémité (20, 26, 27) et de boîtier (31) et dans lequel la séquence présélectionnée est constituée de la séquence extrémité-à-boîtier et anneau-à-boîtier.

5. Système selon la revendication 1, comprenant en outre un moyen (40A) pour détecter une variation notable de l'impédance de dérivation pendant un fonctionnement normal du stimulateur cardiaque et un moyen (84, 98) destiné à déclencher la mesure de l'impédance de dérivation lors d'une détection de la modification significative de l'impédance de dérivation.

6. Système selon la revendication 5, comportant en outre un moyen (80) pour déclencher la mesure de l'impédance de dérivation lors de la survenue d'une modification des dérivations reliées au stimulateur cardiaque (10).

7. Système selon la revendication 6, comportant en outre un moyen (86) destiné à déclencher la mesure de l'impédance de dérivation lors d'une modification de la configuration programmée du stimulateur cardiaque (10).

8. Système selon la revendication 7, comportant en outre un moyen (96) destiné à déclencher la mesure de l'impédance de dérivation lors de la détection d'une absence de capture.

9. Système selon la revendication 1, dans lequel le moyen de détermination (94) comprend un comparateur (94) raccordé pour recevoir des signaux de mesure du moyen (90) de mesure d'impédance de dérivation afin de comparer les signaux à une valeur d'impédance de référence prédéterminée correspondant à une configuration programmée.

*FIG.1*

*FIG.1A*

FIG-2

Fig. 3

EP 0 338 363 B1